(19) Europäisches Patentamt
European Patent Office
Office européen des brevets

(11) EP 2 778 229 A1

(12) # EUROPÄISCHE PATENTANMELDUNG

(43) Veröffentlichungstag:
**17.09.2014 Patentblatt 2014/38**

(51) Int Cl.:
***C12P 41/00*** *(2006.01)* ***C12P 7/42*** *(2006.01)*
***C12N 9/18*** *(2006.01)* ***C12N 9/48*** *(2006.01)*

(21) Anmeldenummer: **13158655.4**

(22) Anmeldetag: **11.03.2013**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder:
- **SANDOZ AG**
  **4056 Basel (CH)**
- **Universität Bielefeld**
  **33615 Bielefeld (DE)**

(72) Erfinder:
- **König, Dr., Burghard**
  **65719 Hofheim (DE)**
- **Wetterich, Dr., Frank**
  **85567 Grafing b. München (DE)**
- **Gröger, Dr., Harald**
  **33739 Bielefeld (DE)**
- **Metzner, Richard**
  **33613 Bielefeld (DE)**

(74) Vertreter: **Vos, Derk**
**Maiwald Patentanwalts GmbH**
**Elisenhof**
**Elisenstraße 3**
**80335 München (DE)**

(54) **Verfahren zur enantioselektiven Herstellung von 3-Hydroxyglutarsäuremonoestern und deren Verwendung**

(57) Die Erfindung betrifft ein Verfahren zur enantioselektiven Herstellung von 3-Hydroxyglutarsäureestern der Formel 1

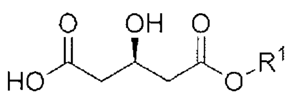

1

,

mit $R^1$ = Alkyl, Aryl, oder substituiertes Alkyl, sowie der Verwendung dieser Verbindungen 1 in der Synthese.

EP 2 778 229 A1

## Beschreibung

Die vorliegende Erfindung betrifft ein Verfahren zur enantioselektiven Herstellung von 3-Hydroxyglutarsäureestern der Formel **1**

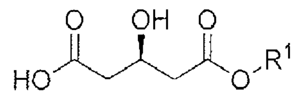

**1**

,

mit $R^1$ = Alkyl, Aryl, oder substituiertes Alkyl, sowie der Verwendung dieser Verbindungen **1** in der Synthese.

Die Verbindungen vom Typ **1** gelten als vielversprechenden Vorstufen für die Herstellung von Rosuvastatin und dessen zugrundeliegendem Alken **6** als typischerweise benötigte Zwischenstufe (Review zu Rosuvastatinsynthesen: Z. Casar, Curr. Org. Chem. 2010, 14, 816-845). Die Herstellung dieses Intermediats **6** ausgehend vom Monoester **1** ist in nachfolgender Abbildung 1 skizziert.

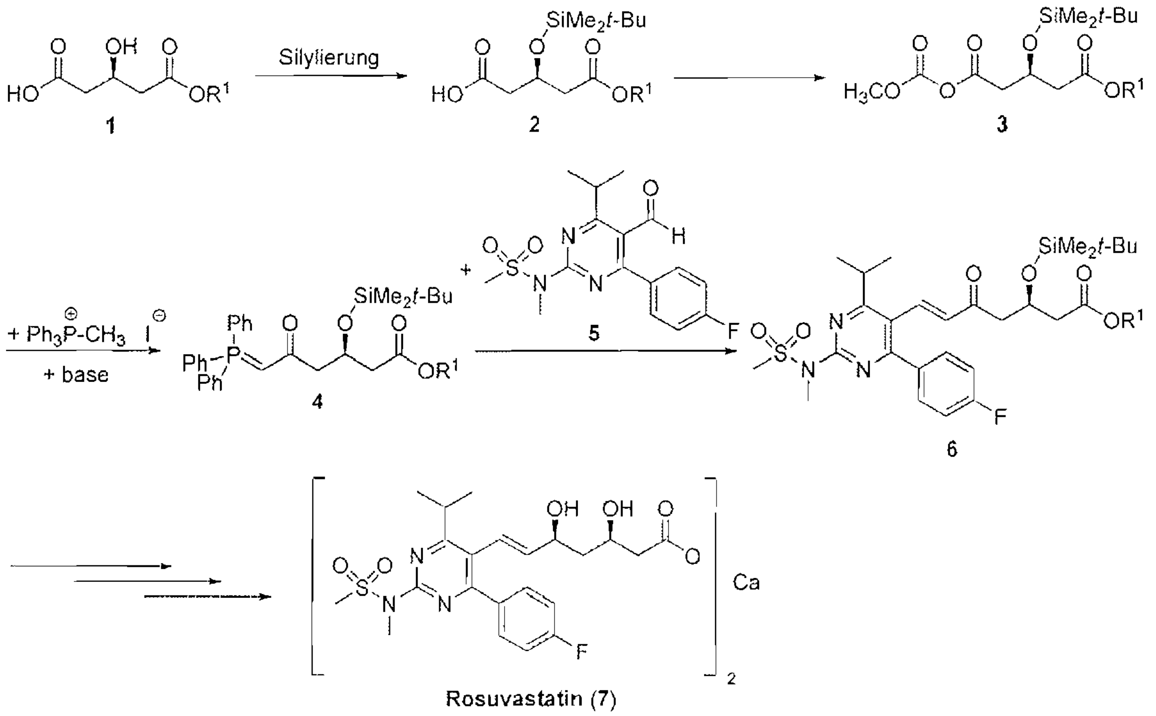


**Abbildung 1.** Verwendung des Monoesters 1 in der Synthese des Phosphor-Ylids **4** als Zwischenstufe für Rosuvastatin (**7**)

Als einfach zugängliche und entsprechend ökonomisch äußerst attraktive Ausgangsverbindungen für die Herstellung der Zielmoleküle vom Typ **1** gelten korrespondierende Diesterverbindungen vom Typ **8** sowie deren *O*-acylierten Derivate vom Typ **9**

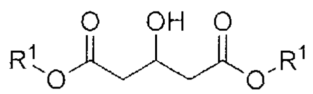

**8**

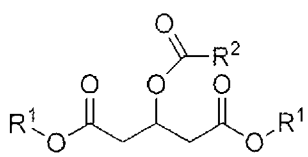

**9**

,

mit $R^1$ = Alkyl, Aryl, oder substituiertes Alkyl, und $R^2$ = -H, Alkyl, , Aryl, substituiertes Alkyl, Aryloxy oder Benzyloxy.

Bislang wird der Verbindungstyp **1** in aufwändiger Weise unter Einsatz stöchiometrischer Mengen chiraler Auxiliare (z.B. (R)-Mandelsäurebenzylester, **11)** und zudem mit mäßigen Raum-Zeit-Ausbeuten ausgehend von 3-Hydroxyglutarsäureanhydrid **10** hergestellt (Abbildung 2).[T. Konoike, Y. Araki, J. Org. Chem. 1994, 59, 7849-7854] Ein weiterer Nachteil dieser bestehenden, vielstufigen Route liegt in der Notwendigkeit, zunächst aus den einfach und gut zugänglichen 3-Hydroxyglutarsäurediestern vom Typ **8** das Anhydrid **10** über mehrere Synthesestufen herzustellen.[C. H. Heathcock, C. R. Hadley, T. Rosen, P. D. Theisen, S. J. Hecker, J. Med. Chem. 1987, 30. 1858-1873]

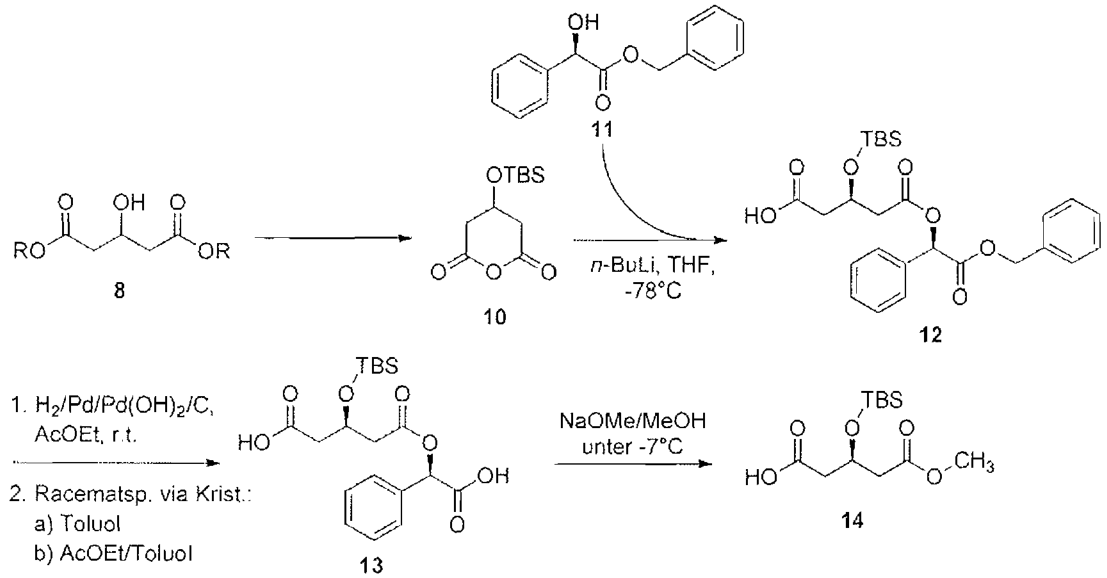


**Abbildung 2.** Synthese des Monoesters **1** aus Anhydrid **10** mit Hilfe eines chiralen Auxiliars

Einen wesentlich attraktiveren Herstellungszugang zum Verbindungstyp **1** würde ein direkter Einsatz der einfach zugänglichen und ökonomisch äußerst attraktiven Diesterverbindungen **8** oder **9** in einem kurzstufigen enantioselektivkatalytischem Syntheseverfahren durch desymmetrisierende enantioselektive Hydrolyse ohne Notwendigkeit der Herstellung eines Anhydrids sowie unter Vermeidung von stöchiometrischen Mengen chiraler Auxiliare darstellen.

Für Vertreter des allgemeinen Diestertyps **8** wurde eine solche Reaktion unter Einsatz von Biokatalysatoren bereits berichtet, allerdings erwies sich bislang deren angestrebte direkte hydrolytische Überführung in Monoester vom Typ **1** als problematisch. So werden beim Einsatz von Esterasen und Lipasen für nicht *O*-acylierte 3-Hydroxyglutarsäurediester vom Typ **2** (mit $R^2$ = H) zwar hohe Enantioselektivitäten von bis zu 95% ee, aber die "falsche" enantiomere (S)-Form erhalten. Beim Einsatz von $\alpha$-Chymotrypsin wird zwar die Bildung der ("richtigen") benötigten absoluten Konfiguration ((*R*)-Form) beobachtet, hierbei aber niedrige, für technische Zwecke ungenügende Enantioselektivitäten erzielt. So führt der Einsatz von $\alpha$-Chymostrpysin in Kombination mit dem entsprechenden Dimethylester lediglich zu einer Enantioselektivität von 76%.[E. E. Jacobsen, B. H. Hoff, A. R. Moen, T. Anthonsen, J. Mol. Cat. B Enzym. 2003, 21, 55-58] Diese geringe Enantioselektivität wurde auch durch eigene Versuche, bei denen unter ähnlichen Reaktionsbedingungen ausgehend vom korrespondierenden Diethylester als Substrat mit $\alpha$-Chymotrypsin ein ee-Wert von nur ca. 60% ee erzielt wurde, bestätigt (Vergleichsversuch, Abbildung 3).

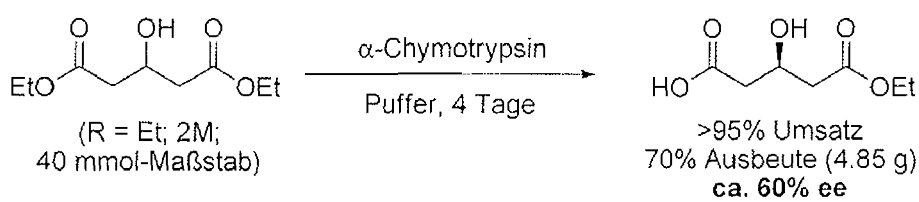


**Abbildung 3.** Hydrolyse von 3-Hydroxyglutarsäurediethylester mit $\alpha$-Chymotrypsin

Entsprechend besitzt dieser Synthesezugang einer "direkten Hydrolyse" von Diestern **8** bei der heutigen technischen Herstellung von Monoester-Verbindungen vom Typ **1** trotz der prinzipiellen offensichtlichen Vorteile der Syntheseroute bislang keine Bedeutung.

Für die alternative Nutzung von Diestern vom Typ **9** in der enzymatischen Hydrolyse wiederum ist bei Verwendung von α-Chmotrypsin eine effiziente, schnell verlaufende und hochenantioselektive Hydrolyse unter Ausbildung des benötigten ("richtigen") (*R*)-Enantiomers beschrieben (Abbildung 4). [R. Öhrlein, G. Baisch, Adv. Synth. Catal. 2003, 345, 713-715 sowie EP 1404642] Allerdings ist bislang kein geeigneter Katalysator sowie generell kein Verfahren für den darauf folgenden notwendigen Reaktionsschritt der selektiven Abspaltung der *O*-Acylgruppe bekannt. Die Eignung eines Enzyms als Katalysator für diesen Schritt erschien hierbei unwahrscheinlich aufgrund der zu erwartenden fehlenden Akzeptanz geladener Substrate (Carboxylate) bei Lipasen und Esterasen sowie aufgrund von zu erwartenden Nebenreaktionen bei Proteasen. Im Detail sind solche Nebenreaktionen die hydrolytische Spaltung der im Molekül enthaltenen Estergruppe -C(O)-OR[1], die aufgrund des kleinen Alkoholrestes bevorzugt verlaufen sollte. Konsequenterweise besteht die industriell entwickelte, allerdings für einen anderen Statin-Wirkstoff geeignete Lösung in einer chemischen Kettenverlängerung des aus dem Diester (vom allgemeinen Typ **9**) gebildeten Hydrolyseprodukt über das korrespondierende Säurechlorid in eine unpolare Verbindung, bei der nun die polare Carboxylatgruppe nicht mehr enthalten ist und entsprechend die Umsetzung dieses hydrophilen Monoesters mit einer Esterase wie erwartet glatt und unter Vermeidung obig genannter Nachteile verläuft und das Zielprodukt ergibt (Abbildung 4).

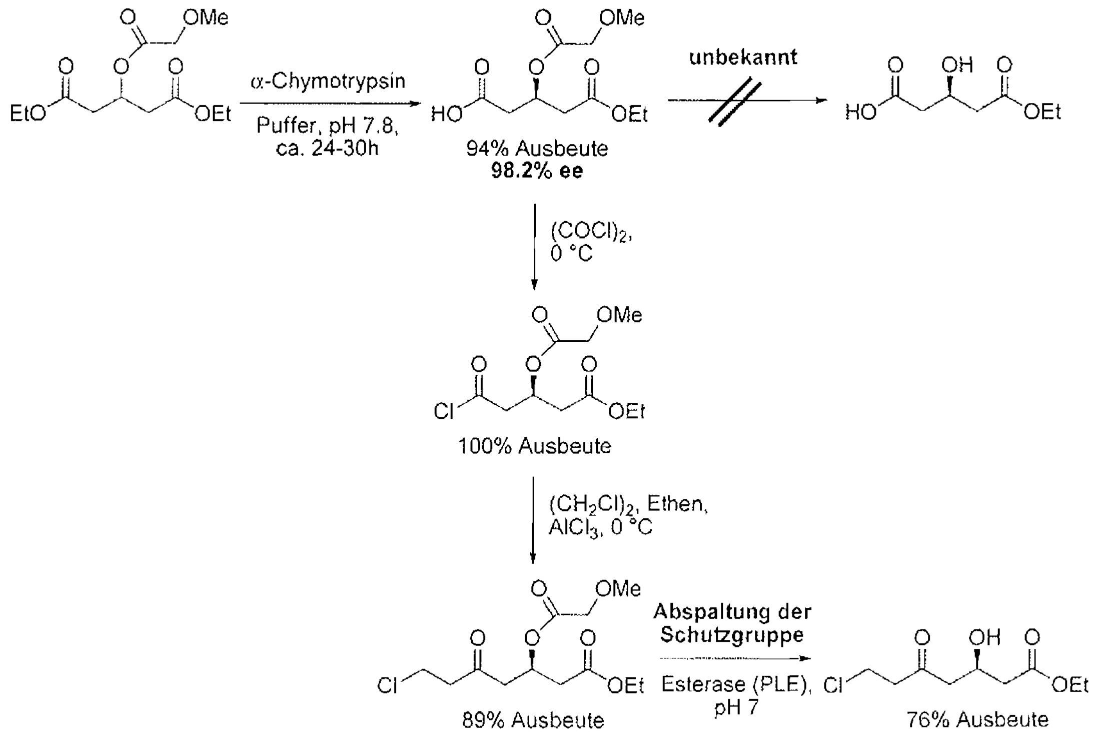


**Abbildung 4.** Effiziente α-Chymotrypsin-katalysierte Diester-Hydrolyse sowie vorgeschaltete Derivatisierung des Reaktionsprodukts vor Abspaltung der Methoxyacetyl-Funktionalität

Allerdings lässt sich eine solche Gesamtverfahrensweise nicht ohne weiteres auf die Herstellung der für das Statin Rosuvastatin benötigten Zwischenstufen übertragen. Entsprechend gilt eine Synthesemethode zur direkten Überführung der Verbindungen vom Typ **9** in Verbindungen vom Typ **1** unverändert als wünschenswert.

Entsprechend bestand die erfindungsgemäße Aufgabe in der Bereitstellung eines Verfahrens zur Herstellung von Verbindungen der oben genannten allgemeinen Formel 1

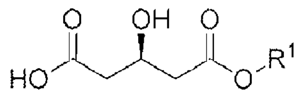

1

,

das obig genannte Nachteile nicht aufweist und in effizienter Weise ausgehend von Verbindungen vom Typ **9**

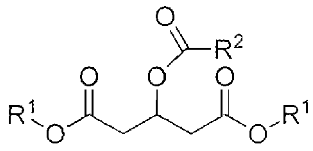

9

,

oder des Typs **15**

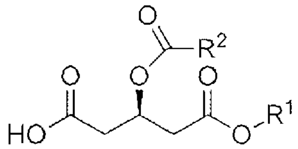

15

ausgeht.

Des weiteren sollte die Notwendigkeit der Herstellung eines Anhydrids sowie der Einsatz von stöchiometrischen Mengen chiraler Auxiliare, wie in der Technik für diese Reaktionen beschrieben, vermieden werden.

Die Aufgabe wurde erfindungsgemäß durch ein Verfahren gelöst, gekennzeichnet dadurch, dass die Hydrolyse der O-Acylbindung einer Verbindung der allgemeinen Formel **15**

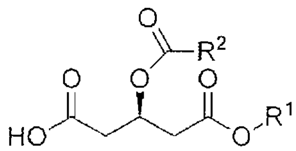

15

,

wobei $R^1$ = Alkyl, Aryl oder ein substituiertes Alkyl ist; und $R^2$ = -H, Alkyl, Aryl, Aryloxy oder Benzyloxy ist, in Gegenwart eines Enzyms (Enzymkomponente 2) durchgeführt wird.

In einem weiteren Aspekt der vorliegenden Erfindung umfasst das Verfahren ferner die Schritte, dass in einem ersten Schritt eine Verbindung der allgemeinen Formel **9**

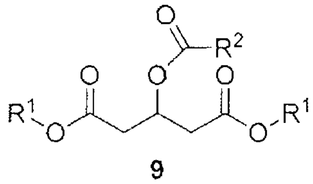

9

,

wobei $R^1$ = Alkyl, Aryl, oder substituiertes Alkyl ist; und
$R^2$ = -H, Alkyl, Aryl, substituiertes Alkyl, Aryloxy oder Benzyloxy ist;
in Gegenwart eines Enzyms (Enzymkomponente 1), bevorzugt hydrolytisch und enantioselektiv, zum korrespondierenden Monoester der allgemeinen Formel **15**

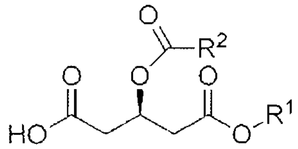

**15**

gespalten wird. Die Verbindung **15** kann optional nach diesem Schritt isoliert werden.

Diese Verbindung **15** kann anschließend, wie oben beschrieben, in Gegenwart eines weiteren Enzyms (Enzymkomponente 2) durch eine Hydrolyse der *O*-Acylgruppe in eine Verbindung der allgemeinen Formel **1**

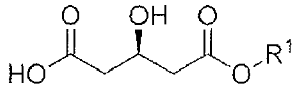

**1**

mit $R^1$ = Alkyl, insbesondere Aryl, substituiertes Alkyl, umgewandelt werden.

Als besonders überraschend kann dabei insbesondere die Abspaltung der O-Acetyl-Gruppe dieser bi-enzymatischen Synthesesequenz, betrachtet werden. So ist die Abspaltung einer *O*-Acetyl-Gruppe in Gegenwart einer einfachen Ester-Gruppe mit Hilfe eines Enzyms so nicht zu erwarten gewesen (siehe auch obige Anmerkungen im Zusammenhang mit Abbildung 4). Vielmehr wäre aufgrund vorhandener Literatur zu erwarten gewesen, dass in Gegenwart von insbesondere Proteasen als Enzymkomponente eher eine Spaltung der verbleibenden OEt-Gruppe statt der *O*-Acetyl-Gruppe erfolgt. So haben Proteasen, die geladene Verbindungen wie deprotonierte Carbonsäuren als Substrate akzeptieren können, eine hohe Tendenz zur hydrolytischen Spaltung einfacher Carbonsäureester wie Methylester und Ethylester und würden entsprechend nicht wie gewünscht die *O*-Acetyl-Gruppe in der Zwischenstufe **15,** sondern die in diesem Molekül ebenfalls noch enthaltene einfachere Alkylester-Einheit (insbesondere bei $R^1$ = Methyl, Ethyl) angreifen. Zugleich wäre in Gegenwart von Lipasen und "klassischen" Esterasen wie die Schweineleberesterase als Alternative zur Gruppe der Hydrolasen gehörenden Enzymkomponenten zu erwarten gewesen, dass keine weitere Abspaltung erfolgt, da es sich beim Substrate für den zweiten Hydrolyseschritt um eine deprotonierte Carbonsäure und entsprechend um eine geladene Verbindung handelt. Für solche Substrate sind die typischerweise sowohl Esterasen als auch Lipasen unreaktiv. Dies wird beispielsweise bei der Nutzung der Schweineleberesterase zur Monohydrolyse von Diestern im wässrigen Reaktionsmedium sichtbar. Die Ursachen, warum in diesem Falle eine enzymatische Hydrolyse in der gewünschten und zugleich überraschenden Art und Weise möglich sind, sind noch unklar und derzeit Gegenstand intensiver Forschungsarbeiten.

In einer besonderen Ausführungsform wird hierbei eine Cephalosporin C-Acetylhydrolase (EC 3.1.1.41) als Enzymkomponente 2 eingesetzt wird. Als bevorzugt haben sich Cephalosporin C-Acetylhydrolasen aus *Bacillus substilis* [F. Knauseder, M. Schiestl, K. Schörgendorfer, US Patent US6465233, 2002.] erwiesen. Die Cephalosporin C-Acetylhydrolase wird vorteilhafterweise in rekombinanter Form eingesetzt. Bei der Enzymformulierung ist sowohl der Einsatz der Cephalosporin C-Acetylhydrolase in "freier" Form als auch in immobilisierter Form [W. Riethorst, A. Reichert, Chimia 1999, 600-607.] sowie in Form eines ggf. rekombinanten Ganzzellkatalysators möglich.

In einer besonderen Ausführungsform des einleitenden Schritts der durch die Enzymkomponente 1 katalysierten Umwandlung des *O*-acylierten Diesters **9** in den *O*-acylierten Monoester **15** (der dann wiederum das Substrat für die obig beschriebene Hydrolyse in Gegenwart der Enzymkomponente 2 zum gewünschten Monoester der Formel **1,** aufweisend eine freie, nicht acylierte Hydroxy-Funktion in 3-Position, darstellt) wird eine Protease eingesetzt. Als besonders vorteilhaft hat sich hierbei der Einsatz von $\alpha$-Chymotrypsin, Trypsin oder Mischungen davon erwiesen. Diese Proteasen können in jeglicher Form eingesetzt werden, beispielsweise als direkt aus natürlichen Quellen isolierte Proteine als auch in rekombinanter Form. Bei der Enzymformulierung ist auch bei der Enzymkomponente 1 deren Einsatz sowohl in "freier" Form als auch in immobilisierter Form möglich. Die Biotransformationen können hierbei beispielsweise sowohl mit gereinigten Proteinen als auch mit Rohextrakten in jeweils nicht-immobilisierter und immobilisierter Form durchgeführt werden. Der Reinheitsgrad der eingesetzten Enzymformulierungen ist hierbei beliebig. Statt dem Einsatz von Enzymen als "zell-freie Enzymformulierungen" ist auch der Einsatz der Enzyme in Form eines ggf. rekombinanten Ganzzellkatalysators (der ebenfalls wiederum in nicht-immobilisierter sowie immobilisierter Form verwendet werden kann) möglich.

Die mit dem erfindungsgemäßen Verfahren hergestellten Verbindungen vom Typ **1** eignen sich in besonderer Weise für die Herstellung von Rosuvastatin (**7**) im Rahmen einer Mehrstufensynthese. Die einzelnen Reaktionen ausgehend von einer Verbindung vom Typ **2** bis zum gewünschten Rosuvastatin (**7**) sind dabei bereits im Detail in der Literatur beschrieben (Review zu Rosuvastatinsynthesen: Z. Casar, Curr. Org. Chem. 2010, 14, 816-845). Entsprechend erfolgt nach dem Bereitstellen einer mit Hilfe des erfindungsgemäßen Verfahren hergestellten Verbindung **1,** wobei $R^1$

= Alkyl, Aryl, oder substituiertes Alkyl ist, zunächst eine Umsetzung von **1** zur *O*-silylierten Verbindung **2**

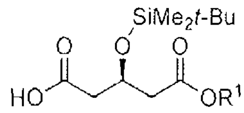

**2**

unter Einsatzes eines Silylierungsreagenzies der Formel X-$SiMe_2$*t*-Bu, wobei X = Cl, Br ist, gefolgt von einer Transformation der Verbindung **2** zur Anhydrid **3**

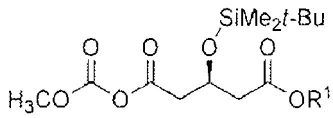

**3**

unter Einsatz von Chlorameisensäuremethylester. Der Anhydrid **3** reagiert dann durch Reaktion mit Methyltriphenylphosphoniumbromid in bewährter Weise zum Ylid **4**

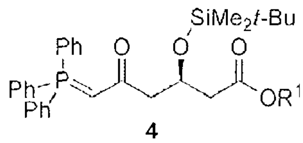

**4**

Anschließend gibt eine Wittig-Reaktion der Verbindung **4** mit dem Aldehyd **5**

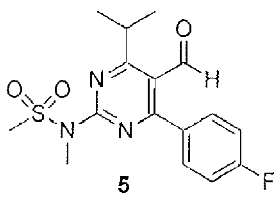

**5**

die Verbindung **6**

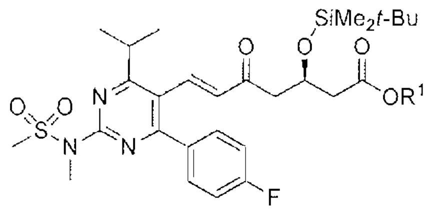

**6**

die abschließend in bewährter Weise nach literaturbekannten Verfahren via Hydrolyse, Reduktion und Neutralisation mit einem basischen Calciumsalz in das gewünschte Rosuvastatin (**7**) oder Salz davon

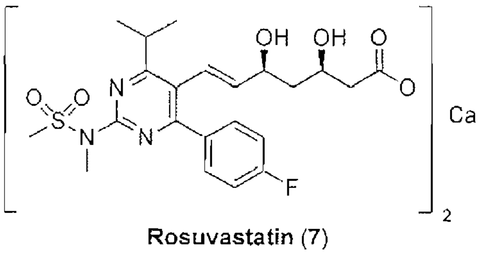

**Rosuvastatin** (7)

umgewandelt wird. Die gebildeten Zwischenstufen können hierbei vor der weiteren Reaktionsstufe isoliert und gereinigt werden. Alternativ kann aber auch auf eine Isolierung und Reinigung der Zwischenstufen verzichtet werden und die gebildeten Reaktionsgemische bzw. nach Extraktion und optional Entfernen des Lösungsmittelkomponente resultierenden Rohprodukte, enthaltend die gewünschte Zwischenstufe, können direkt für in der nächsten Reaktionsstufe eingesetzt werden.

Eine besondere Ausführungsform der Erfindung umfasst ein Verfahren zur Herstellung von Rosuvastatin (**7**) **dadurch gekennzeichnet, dass** eine Verbindung **1,** hergestellt nach einem oben beschriebenen Verfahren, zu Rosuvastatin (**7**) umgesetzt wird.

In einer weiteren besonderen Ausführungsform umfasst das Verfahren zur Herstellung von Rosuvastatin (**7**), die folgenden Syntheseschritte:

a) Bereitstellen einer Verbindung **1**, wobei $R^1$ = Alkyl, Aryl, oder substituiertes Alkyl ist, hergestellt nach dem erfindungsgemäßen Verfahren, wie oben beschrieben"

b) Umsetzen der Verbindung **1** unter Einsatz von *t*-Butyldimethylchlorsilan ($Me_2$(*t*-Bu)SiCl) zur silylierten Verbindung **2**

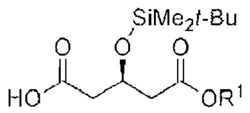

**2**

,

c) Umsetzen der Verbindung **2** unter Einsatz von Chlorameisensäuremethylester zur Verbindung **3**

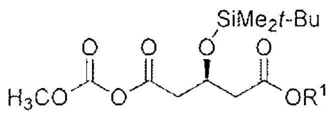

**3**

,

d) nachfolgendes Umsetzen der Verbindung **3** unter Einsatz von Methyltriphenylphosphoniumhalogenid wie z.B, -bromid und / oder -chlorid zur Verbindung **4**

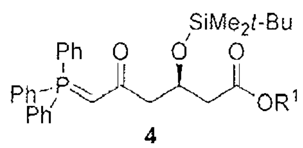

**4**

,

e) nachfolgendes Umsetzen der Verbindung **4** mit der Verbindung **5**

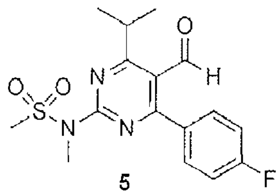

5

in einer Wittig-Reaktion zur Verbindung **6**

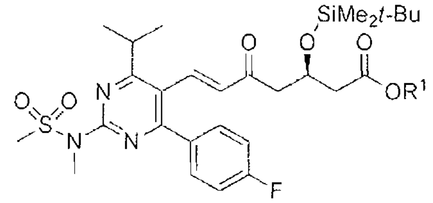

6

und

f) nachfolgendes Umsetzen der Verbindung **6** zu Rosuvastatin (**7**) oder einem Salz davon.

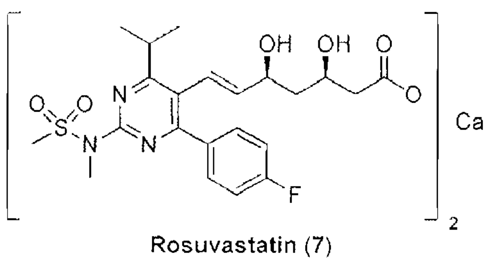

Rosuvastatin (7)

In einer besonderen Ausführungsform werden die als Zwischenstufe gebildete Verbindung **15** und / oder **1** und / oder **2** und / oder **3** und / oder **4** und / oder **6** nicht isoliert, sondern die diese jeweilige Verbindung **15** und / oder **1** und / oder **2** und / oder **3** und / oder **4** und / oder **6** enthaltene Reaktionslösung direkt *in situ* ohne Aufarbeitung und Reinigung weiter für den darauffolgenden Syntheseschritt eingesetzt wird.

Der Begriff "Alkyl", wie hierin verwendet, beschreibt einen gradkettigen oder verzweigten Kohlenwasserstoff mit 1, 2, 3, 4, 5, 6, 7, 8, 9, oder 10 Kohlenstoffatomen (-($C_1$-$C_{10}$)Alkyl). Typische -($C_1$-$C_{10}$)Alkyle sind Methyl, Ethyl, *n*-Propyl, *i*-Propyl, *n*-Butyl, *sec*-Butyl, *tert*-Butyl, 3-Pentyl, *n*-Hexyl, *n*-Heptyl, *n*-Octyl, *n*-Nonyl und *n*-Decyl. In einer Ausführungsform werden die Alkyle ausgewählt aus gradkettigen oder verzweigten - ($C_1$-$C_4$)Alkylen. Typische -($C_1$-$C_4$)Alkyle sind Methyl, Ethyl, *n*-Propyl, *i*-propyl, *n*-Butyl, *sec*-Butyl, oder *tert*-Butyl.

Der Begriff "Aryl" wie hierin verwendet, beschreibt -($C_6$-$C_{14}$)Aryle. Typische Beispiele für -($C_6$-$C_{14}$)Aryle sind Phenyl, Naphthalenyl, Phenanthryl, Indenyl oder dergleichen. Bevorzugt sind -($C_6$-$C_{14}$)Aryle. Ein besonders bevorzugtes Aryl is Phenyl.

Der Begriff "substituiertes Alkyl", wie hierin verwendet, beschreibt einen gradkettigen oder verzweigten Kohlenwasserstoff mit 1, 2, 3, 4, 5, 6, 7, 8, 9, oder 10 Kohlenstoffatomen (-($C_1$-$C_{10}$)Alkyl), bei dem ein oder mehrere Wasserstoffatome des Kohlenwasserstoffs durch andere Substituenten ersetzt wurden. Typische Substituenten sind Halogen (d.h. -F, -Cl, -Br, oder -I), Phenyl, -O($C_1$-$C_{10}$)Alkyl, oder dergleichen. Bevorzugte substituierte Alkyle sind z.B. Chlormethyl, Brommethyl, Iodmethyl, Methoxymethyl, Ethoxymethyl, oder Benzyl. Der Begriff "Benzyloxy", wie hierin verwendet, bedeutet -O-$CH_2$-Phenyl.

Der Begriff "Aryloxy", wie hierin verwendet, bedeutet, das eine der oben genannten Aryl-Gruppen über ein Sauerstoff gebunden ist, -O-Aryl. Ein typisches Beispiel für eine Aryloxy-Gruppe ist Phenoxy, i.e. -O-Ph.

Der Begriff, dass ein Enzym in "freier Form" eingesetzt wird, bedeutet im Zusammenhang mit der Erfindung, dass die Enzyme nicht immobilisiert sind. Dies schließt ebenfalls den Einsatz der Enzyme als Ganzzellkatalysatoren ein, d.h. den Einsatz von Zellen, die die gewünschten Enzyme enthalten.

Der Begriff "enantioselektiv" wie hierin verwendet bedeutet, dass bei der Umsetzung eines prochiralen Eduktes eines der beiden möglichen Enatiomere bevorzugt ausgebildet wird. Der Enantiomerenüberschuss ("enantiomeric excess" oder "ee") wird als Maß für die Enantioselektivität angegeben:

$$\% \text{ ee} = \left[ \frac{\text{Hauptenantiomer (mol) - Nebenenantiomer (mol)}}{\text{Hauptenantiomer (mol) + Nebenenantiomer (mol)}} \right] \text{ x } 100\%$$

Bei den Darstellungen der Verbindungen, wie z.B. der Verbindung **1** oder **15,** ist die jeweils bevorzugte absolute Konfiguration gezeigt. Die weiteren möglichen Konfigurationen sind ebenfalls von der Offenbarung umfasst.

Der Begriff "Salz" wie hierin verwendet bezieht sich insbesondere auf ein Salz, das durch eine saure oder basische funktionale Gruppe gebildet werden kann. Typische Beispiele schließen Sulfate, Citrate, Acetate, Chloride, Bromide, Iodide, Nitrate, Phosphate, Sulfonate oder dergleichen ein. Ferner schließen Salze der Verbindungen, die eine saure funktionelle Gruppe, wie z.B. eine Carbonsäure-Gruppe aufweisen Salze mit akzeptablen anorganischen und organischen Basen ein. Geeignete Basen schließen z.B. Hydroxide der Alkalimetalle wie z.B. Natrium, Kalium, Cesium oder Lithium ein; Hydroxide der Erdalkalimetalle wie z.B. Calcium oder Magnesium oder Hydroxide weiterer Metalle ein.

**Experimentelle Beispiele:**

**Beispiel 1: Acetylierung von 3-Hydroxyglutarsäurediethylester**

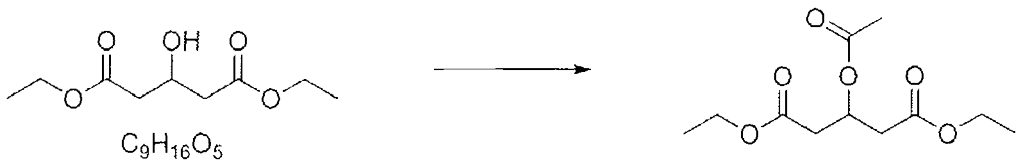

$C_9H_{16}O_5$
M = 204.22 g/mol

$C_{11}H_{18}O_6$
M = 246.26 g/mol

Essigsäureanhydrid (19,9mL, 210 mmol, 1.05 Äq.) wird mit Zinkperchlorat-Hexahydrat (78.2 mg, 0.21 mmol, 0.10 mol%) versetzt und für 10 Minuten bei Raumtemperatur gerührt. Die klare, farblose Lösung wird mit 3-Hydroxyglutarsäurediethylester (37.0 mL, 200 mmol) versetzt und für weitere vier Stunden bei Raumtemperatur gerührt. Destilliertes Wasser (50 mL) wird hinzugefügt und die Emulsion für weitere 15 Minuten bei Raumtemperatur gerührt. Nach Zugabe von *tert*-Butylmethylether (MTBE, 100 mL) werden die Phasen getrennt und die organische Phase noch dreimal mit gesättigter $NaHCO_3$-Lösung (je 100 mL) gewaschen. Die organische Phase wird über $MgSO_4$ getrocknet und das Lösungsmittel *in vacuo* vollständig entfernt. Man erhält 3-Acetoxyglutarsäurediethylester (47.9 g, 195 mmol, 97.5%) als farblose, viskose Flüssigkeit.

**Beispiel 2: Desymmetrisierung des prochiralen 3-Acetoxyglutarsäurediethylesters mit Hilfe einer Hydrolase (mit Isolation des Zwischenprodukts)**

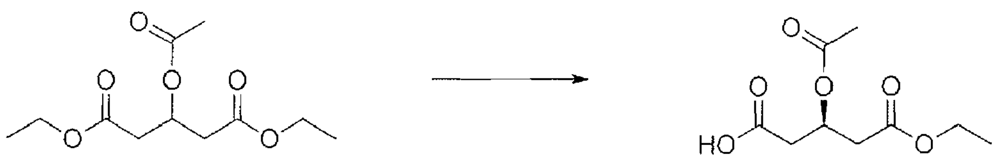

$C_{11}H_{18}O_6$
M = 246.26 g/mol

$C_9H_{14}O_6$
M = 218.20 g/mol

3-Acetoxyglutarsäurediethylester (24.6 g, 100.0 mmol) wird unter starker Durchmischung mit einem Propellerrührer in Phosphatpuffer (15.0 mL, 50mM, pH 8.0) emulgiert. Eine Enzymformulierung aus Chymotrypsin/Trypsin im

Verhältnis 1:1 (1.5 g, Biozym CHY-04) wird in Phosphatpuffer (10.0 mL, 50mM, pH 7.0) gelöst und der pH-Wert mit 4.0 M NaOH auf 8.0 eingestellt. Nach Zugabe dieser Enzymlösung zur Substratmischung wird die resultierende Emulsion für ca. 27 Stunden (Zeitpunkt des stöchiometrischen Titerverbrauchs) bei Raumtemperatur gerührt und der pH-Wert über einen pH-Stat (Titrino, 4.0 M NaOH) konstant auf pH 8.0 +- 0.3 gehalten. Hierbei wird die entstehende Carbonsäure mit 4.0 M NaOH neutralisiert und über deren Verbrauch der Reaktionsverlauf verfolgt. Anschließend wird die Reaktionslösung mit festem Natriumchlorid gesättigt und mit konz. HCl auf ca. pH 1-2 eingestellt. Die denaturierte Hydrolase wird über eine Glasfilterfritte abgetrennt und das Filtrat zweimal mit Ethylacetat (je 100 mL) extrahiert. Die organische Phase wird über $MgSO_4$ getrocknet und das Lösungsmittel am Rotationsverdampfer entfernt. Man erhält *(3R)*-Acetoxy-5-ethoxy-5-oxopentansäure (21.4 g, 98.1 mmol, 98.1%) als farblose, viskose Flüssigkeit. Eine ee-Wert-Analyse über die *(S)*-1-Phenylethylamin-Derivate (Daicel ChiralPak OJ-H, SC-$CO_2$/*i*-PrOH 95:5 (v/v), 0.8 mL/min, 10 MPa Rückdruck, 220 nm) zeigt einen Diastereomerenüberschuss von 97.5% de, entsprechend einem Enantiomerenüberschuss von 97.5% ee.

**Beispiel 3: Deacetylierung des enantiomerenangereicherten Hydro-*(3R)*-Acetoxyglutar-säuremonoethylesters mit Hilfe einer Hydrolase (mit Isolation des Zwischenprodukts)**

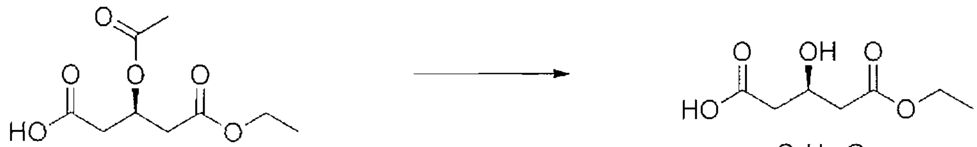

$C_9H_{14}O_6$
M = 218.20 g/mol

$C_7H_{12}O_5$
M = 176.17 g/mol

*(3R)*-Acetoxy-5-ethoxy-5-oxopentansäure (20.7 g, 95.0 mmol) wird in Phosphatpuffer (24 mL, 50 mM, pH 8.0) emulgiert und der pH-Wert mit 4.0 M NaOH auf 8.0 eingestellt. Nach Zugabe von immobilisierter Cephalosporin C Acetylhydrolase (1.0 g, erhalten nach W. Riethorst, A. Reichert, Chimia 1999, 600-607) wird die Lösung bei Raumtemperatur gerührt und der pH-Wert über einen pH-Stat (Titrino, 4.0 M NaOH) konstant gehalten. Hierbei wird die entstehende Carbonsäure mit 4.0 M NaOH neutralisiert und über deren Verbrauch der Reaktionsverlauf verfolgt. Der Reaktionsverlauf zeigt einen vollständigen Umsatz nach ca 26 Stunden. Anschließend wird die Reaktionslösung mit festem Natriumchlorid gesättigt, mit konz. HCl auf ca. pH 1-2 eingestellt und dreimal mit Ethylacetat (je 75 mL) extrahiert. Die vereinigten organischen Phasen werden über $MgSO_4$ getrocknet und das Lösungsmittel am Rotationsverdampfer vollständig entfernt. Man erhält *(3R)*-5-Ethoxy-3-hydroxy-5-oxopentansäure (13.9 g, 78.9 mmol, 83.0% Ausbeute) als farblose, viskose Flüssigkeit.

**Beispiel 4: Bi-enzymatischer, sequentieller Eintopfprozess zur Synthese von *(3R)*-5-Ethoxy-3-hydroxy-5-oxo-pentansäure**

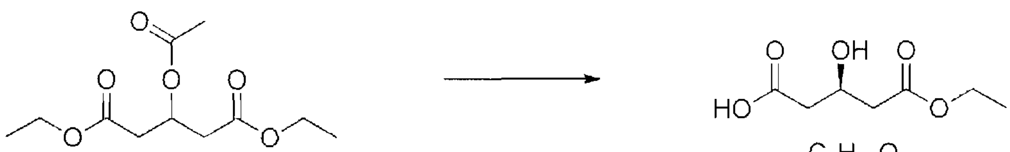

$C_{11}H_{18}O_6$
M = 246.26 g/mol

$C_7H_{12}O_5$
M = 176.17 g/mol

3-Acetoxyglutarsäurediethylester (49.2 g, 200.0 mmol) wird unter starker Durchmischung mit einem Propellerrührer in Phosphatpuffer (30.0 mL, 50mM, pH 8.0) emulgiert. Eine Enzymformulierung aus Chymotrypsin/Trypsin im Verhältnis 1:1 (3.0 g, Biozym CHY-04) wird in Phosphatpuffer (20.0 mL, 50mM, pH 7.0) gelöst und der pH-Wert mit 4.0 M NaOH auf 8.0 eingestellt. Nach Zugabe dieser Enzymlösung zur Substratmischung wird die resultierende Emulsion für ca. 27 Stunden (Zeitpunkt des stöchiometrischen Titerverbrauchs) bei Raumtemperatur gerührt und der pH-Wert über einen pH-Stat (Titrino, 4.0 M NaOH) konstant auf pH 8.0 +- 0.3 gehalten. Hierbei wird die entstehende Carbonsäure

mit 4.0 M NaOH neutralisiert und über deren Verbrauch der Reaktionsverlauf verfolgt. Anschließend wird die homogene Reaktionslösung in eine Ultrafiltrations-Rührzelle überführt und bei 800 UpM und 2.0 bar Argon-Überdruck über eine Ultrafiltrationsmembran (MWC 10.000 Da) vom homogen gelösten Chymotrypsin/Trypsin-Gemisch getrennt. Das Filtrat wird mit immobilisierter Cephalosporin C Acetylhydrolase (2.0 g) versetzt und die Suspension bei Raumtemperatur gerührt. Der pH-Wert wird über einen pH-Stat (Titrino, 4.0 M NaOH) konstant auf pH 8.0 +- 0.3 gehalten. Hierbei wird erneut die entstehende Carbonsäure mit 4.0 M NaOH neutralisiert und über deren Verbrauch der Reaktionsverlauf verfolgt. Der Reaktionsverlauf zeigt einen vollständigen Umsatz nach ca. 26 Stunden. Anschließend wird die Suspension über eine Glasfilterfritte (Por 4) von dem immobilisierten Enzym getrennt. Das Filtrat wird mit festem Natriumchlorid gesättigt, mit konz. HCl auf ca. pH 1-2 eingestellt und anschließend dreimal mit Ethylacetat (je 100 mL) extrahiert. Die vereinigten organischen Phasen werden über $MgSO_4$ getrocknet und das Lösungsmittel am Rotationsverdampfer vollständig entfernt. Man erhält *(3R)*-5-Ethoxy-3-hydroxy-5-oxopentansäure (33.1 g, 188 mmol, 94% Ausbeute) als fahlrote, viskose Flüssigkeit. Über erneute Acetylierung und Derivatisierung mit *(S)*-1-Phenylethylamin wurde eine Selektivität von 98.1% ee (Daicel ChiralPak OJ-H, SC-$CO_2$/*i*-PrOH 95:5, 0.8 mL/min, 10 MPa Rückdruck, 220 nm) bestimmt.

**Patentansprüche**

**1.** Verfahren zur Herstellung einer Verbindung der allgemeinen Formel **1**

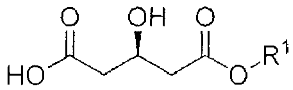

**1**

,

wobei $R^1$ = Alkyl, Aryl oder ein substituiertes Alkyl ist,
umfassend die Reaktion einer Verbindung der allgemeinen Formel **15**

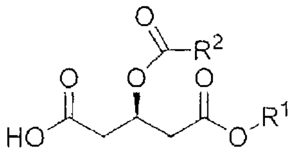

**15**

,

wobei $R^1$ = Alkyl, Aryl oder ein substituiertes Alkyl ist; und $R^2$ = -H, Alkyl, Aryl, substituiertes Alkyl, Aryloxy oder Benzyloxy ist,
**dadurch gekennzeichnet, dass** die Hydrolyse der O-Acylbindung in Gegenwart eines Enzyms (Enzymkomponente 2) durchgeführt wird.

**2.** Verfahren nach Anspruch 1, ferner umfassend die folgenden Schritte:

a) Reaktion der Verbindung der Formel **9**

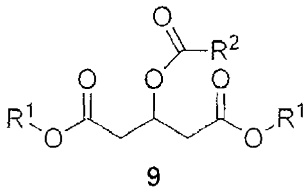

**9**

in Gegenwart eine Enzyms (Enzymkomponente 1) hydrolytisch zum Monoester der allgemeinen Formel **15,**

wobei $R^1$ = Alkyl, Aryl, oder substituiertes Alkyl ist; und
$R^2$ = -H, Alkyl, Aryl, substituiertes Alkyl, Aryloxy oder Benzyloxy ist;
b) Optional Isolierung der Verbindung **15.**

**3.** Verfahren nach Anspruch 1, **dadurch gekennzeichnet, dass** die Enzymkomponente 2 eine Acylhydrolase umfasst.

**4.** Verfahren nach Anspruch 1 bis 2, **dadurch gekennzeichnet, dass** eine Acetylhydrolase als Enzymkomponente 2 eingesetzt wird.

**5.** Verfahren nach einem der Ansprüche 1 bis 4, **dadurch gekennzeichnet, dass** eine Cephalosporin C-Acetylhydrolase (EC 3.1.1.41) als Enzymkomponente 2 eingesetzt wird.

**6.** Verfahren nach einem der Ansprüche 1 bis 5, **dadurch gekennzeichnet, dass** als Enzymkomponente eine Cephalosporin C-Acetylhydrolase aus *Bacillus substilis* eingesetzt wird.

**7.** Verfahren nach einem der Ansprüche 1 bis 6, **dadurch gekennzeichnet, dass** Wasser, optional mit einer Pufferkomponente bzw. Salzen versetzt, als Reaktionsmedium eingesetzt wird.

**8.** Verfahren nach einem der Ansprüche 1 bis 7, **dadurch gekennzeichnet, dass** die Umsetzungen des Gesamtverfahrens mit volumetrischen Substratmengen (Substratkonzentrationen) von >100 mM, vorzugsweise >1 M und mehr bevorzugt >2 M durchgeführt werden.

**9.** Verfahren nach einem der Ansprüche 1 bis 8, **dadurch gekennzeichnet, dass** die Verbindung **9** und / oder **15** als Substratkomponente in der jeweiliger Reaktionsstufe

(a) über einen bestimmten Zeitraum zudosiert wird;
(b) in gesamter Menge von Anfang an vorgelegt wird; oder
(c) eine Menge vorgelegt wird und die Restmenge anschließend zudosiert wird.

**10.** Verfahren nach einem der Ansprüche 1 bis 9, **dadurch gekennzeichnet, dass** eine Protease als Enzymkomponente 1 eingesetzt wird.

**11.** Verfahren nach Anspruch 10, **dadurch gekennzeichnet, dass** die Protease $\alpha$-Chymotrypsin, Trypsin oder Mischungen davon umfasst.

**12.** Verfahren nach einem der Ansprüche 1 bis 11, **dadurch gekennzeichnet, dass** die Enzyme in freier Form eingesetzt werden.

**13.** Verfahren zur Herstellung von Rosuvastatin (**7**) **dadurch gekennzeichnet,**

a) Bereitstellen einer Verbindung **1**, hergestellt nach einem der Verfahren der Ansprüche 1 bis 12;
b) Umsetzen der Verbindung **1** zu Rosuvastatin (**7**) oder einem Salz davon.

**14.** Verfahren nach einem der Ansprüche 1 bis 13, wobei $R^1$ = Methyl, Ethyl, n-Propyl, i-Propyl, n-Butyl, sec-Butyl, tert-Butyl, Phenyl oder Benzyl ist.

**15.** Verfahren nach einem der Ansprüche 1 bis 14, wobei $R^2$ = Methyl, Ethyl, n-Propyl, i-Propyl, n-Butyl, sec-Butyl, tert-Butyl, Chlormethyl, Methoxymethyl, Phenyl oder Benzyl ist.

Europäisches Patentamt
European Patent Office
Office européen des brevets

# EUROPÄISCHER RECHERCHENBERICHT

Nummer der Anmeldung
EP 13 15 8655

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | ÖHRLEIN R ET AL: "Chemo-Enzymatic Approach to Statin Side-Chain Building Blocks", ADVANCED SYNTHESIS AND CATALYSIS, WILEY, WEINHEIM, DE, Bd. 345, 1. Januar 2003 (2003-01-01), Seiten 713-715, XP002388465, ISSN: 1615-4169, DOI: 10.1002/ADSC.200303028 * das ganze Dokument * ----- | 1-15 | INV. C12P41/00 C12P7/42 C12N9/18 C12N9/48 |
| A | WO 03/004450 A1 (CIBA SC HOLDING AG [CH]; OEHRLEIN REINHOLD [DE]; BAISCH GABRIELE [DE];) 16. Januar 2003 (2003-01-16) * das ganze Dokument * ----- | 1-15 | |
| A | WO 2011/106546 A1 (TEVA PHARMA [IL]; TEVA PHARMA [US]; PAAL TIHAMER [HU]; TOTH LASZLO [HU]) 1. September 2011 (2011-09-01) * das ganze Dokument * ----- | 1-15 | RECHERCHIERTE SACHGEBIETE (IPC) |
| A | JACOBSEN E E ET AL: "Enantioselective enzymatic preparation of chiral glutaric monocarboxylic acids and amides", JOURNAL OF MOLECULAR CATALYSIS. B, ENZYMATIC, ELSEVIER, AMSTERDAM, NL, Bd. 21, 1. Januar 2003 (2003-01-01), Seiten 55-58, XP002648570, ISSN: 1381-1177 * das ganze Dokument * ----- -/-- | 1-15 | C12P C12N |

Der vorliegende Recherchenbericht wurde für alle Patentansprüche erstellt

1

| Recherchenort | Abschlußdatum der Recherche | Prüfer |
|---|---|---|
| Berlin | 31. Juli 2013 | Schneider, Patrick |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 13 15 8655

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | RIISE MOEN A ET AL: "Absolute configurations of monoesters produced by enzyme catalyzed hydrolysis of diethyl 3-hydroxyglutarate", TETRAHEDRON ASYMMETRY, PERGAMON PRESS LTD, OXFORD, GB, Bd. 15, Nr. 10, 24. Mai 2004 (2004-05-24), Seiten 1551-1554, XP004508430, ISSN: 0957-4166, DOI: 10.1016/J.TETASY.2004.04.007 * das ganze Dokument * ----- | 1-15 | |
| | | | RECHERCHIERTE SACHGEBIETE (IPC) |

Der vorliegende Recherchenbericht wurde für alle Patentansprüche erstellt

1

| Recherchenort | Abschlußdatum der Recherche | Prüfer |
|---|---|---|
| Berlin | 31. Juli 2013 | Schneider, Patrick |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 13 15 8655

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

31-07-2013

| Im Recherchenbericht angeführtes Patentdokument | Datum der Veröffentlichung | Mitglied(er) der Patentfamilie | Datum der Veröffentlichung |
|---|---|---|---|
| WO 03004450 A1 | 16-01-2003 | AR 034733 A1 | 17-03-2004 |
| | | AR 034734 A1 | 17-03-2004 |
| | | AR 034735 A1 | 17-03-2004 |
| | | BR 0210842 A | 13-07-2004 |
| | | CA 2448917 A1 | 16-01-2003 |
| | | CN 1610657 A | 27-04-2005 |
| | | CN 101880227 A | 10-11-2010 |
| | | CZ 20040150 A3 | 15-09-2004 |
| | | EP 1404639 A1 | 07-04-2004 |
| | | EP 2161250 A1 | 10-03-2010 |
| | | ES 2340260 T3 | 01-06-2010 |
| | | HR P20040119 A2 | 30-04-2005 |
| | | HU 0401145 A2 | 28-09-2004 |
| | | JP 2004533479 A | 04-11-2004 |
| | | KR 20040017277 A | 26-02-2004 |
| | | KR 20040017278 A | 26-02-2004 |
| | | PL 366392 A1 | 24-01-2005 |
| | | PT 1404642 E | 29-03-2010 |
| | | US 2004186313 A1 | 23-09-2004 |
| | | US 2008312466 A1 | 18-12-2008 |
| | | US 2011046411 A1 | 24-02-2011 |
| | | WO 03004450 A1 | 16-01-2003 |
| WO 2011106546 A1 | 01-09-2011 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1404642 A
- US 6465233 B, F. Knauseder, M. Schiestl, K. Schörgendorfer


### In der Beschreibung aufgeführte Nicht-Patentliteratur


- **Z. CASAR.** *Curr. Org. Chem.,* 2010, vol. 14, 816-845
- **T. KONOIKE ; Y. ARAKI.** *J. Org. Chem.,* 1994, vol. 59, 7849-7854
- **C. H. HEATHCOCK ; C. R. HADLEY ; T. ROSEN ; P. D. THEISEN ; S. J. HECKER.** *J. Med. Chem,* 1987, vol. 30, 1858-1873
- **E. E. JACOBSEN ; B. H. HOFF ; A. R. MOEN ; T. ANTHONSEN.** *J. Mol. Cat. B Enzym.,* 2003, vol. 21, 55-58
- **R. ÖHRLEIN ; G. BAISCH.** *Adv. Synth. Catal.,* 2003, vol. 345, 713-715
- **W. RIETHORST ; A. REICHERT.** *Chimia,* 1999, 600-607